# EUROPÄISCHE PATENTSCHRIFT

(11) **EP 4 363 916 B1**
(45) Veröffentlichungstag und Bekanntmachung des Hinweises auf die Patenterteilung: **04.06.2025**
(21) Anmeldenummer: 22753596.0
(22) Anmeldetag: 21.06.2022
(51) Int. Cl.: G02B 19/00, F24F 8/22, G02B 27/09, G02B 5/124

(54) **VORRICHTUNG ZUR LICHTVERSTÄRKUNG MITTELS REFLEXIONEN IN LEITUNGEN**
DEVICE FOR LIGHT AMPLIFICATION BY MEANS OF REFLECTIONS IN LINES
DISPOSITIF D'AMPLIFICATION DE LUMIÈRE PAR RÉFLEXION DANS DES CONDUITES

(30) Priorität: 30.06.2021 DE 102021003356
(43) Veröffentlichungstag der Anmeldung: 08.05.2024
(73) Patentinhaber: Milosiu, Johann-Marius, 91058 Erlangen (DE)
(72) Erfinder: Milosiu, Johann-Marius, 91058 Erlangen (DE)
(86) Internationale Anmeldenummer: PCT/DE2022/000068
(87) Internationale Veröffentlichungsnummer: WO 2023/274438

(56) Entgegenhaltungen:
- EP-A1- 3 378 501
- CN-A- 109 489 366
- CN-A- 111 708 159
- DE-A1- 102005 032 934
- DE-A1- 102018 221 634
- DE-A1- 102020 002 104
- DE-A1- 102020 003 124
- DE-A1- 102021 000 458
- DE-U1- 202021 100 238

## Beschreibung

Die Erfindung bezieht sich auf eine Vorrichtung zur Lichtverstärkung mittels multiplen Reflexionen in Leitungen.

Vorrichtungen die einem ähnlichen Zweck dienen, sind recht bekannt. So gibt es Kanäle oder Leitungen für Fluide, welche verspiegelte Wände aufweisen. Sie dienen dazu, die Strahlung von Lichtquellen durch Reflexion länger in den Leitungen und den darin enthaltenen Medium wirken zu lassen. So ist es z.B. DE 20 2021 100 238 U1 oder EP 3 378 501 A1. Der Nachteil dieser Art von Verspiegelung liegt darin, daß nach wenigen Reflexionen die Strahlung sich in axialer Richtung verflüchtigt und die gewünschten Effekte, wie z.B. die Intensivierung der Strahlung bei der Desinfektion der Luft aus den Leitungen durch UV-Strahlung gemindert wird und eine größere Leistung und Anzahl von UV-Lichtquellen erforderlich ist. Aus DE 17 64 878 A und DE 12 04 747 A sind jeweils auf dem Gebiet der Lasertechnik eingesetzte Vorrichtungen sowie Verfahren zur Lichtverstärkung bekannt, bei denen Licht mehrfach von Wänden reflektiert wird, und dabei innerhalb einer Festkörper-Leitung in Umläufen immer wieder den selben Weg innerhalb eines abgegrenzten Bereichs nimmt. Dabei können diese Vorrichtungen nur intrinsische und streng parallele oder kohärente Strahlen verstärken und kommen als Verstärker für divergente Licht- und insbesondere UV-Strahler nicht in Frage.

Die vorliegende Erfindung umfasst in einem Ausführungsbeispiel, eine spezielle Anordnung von parallel angeordneten Spiegelpaaren, die in 90° Winkel zueinander stehen, wobei jeder Spiegel im 45° Winkel zur Achse steht und nur an einer der vier Leitungswänden angebracht sind, während die anderen Wände einfach verspiegelt sind, zu verwenden. Diese Spiegelpaare sind quer auf die Längsachse der jeweiligen Leitung. orientiert. Dieses System von Spiegeln ermöglicht auch die Strahlung von kleinflächigen und relativ stark divergierenden Lichtstrahlern, wie z.B. UV-Leuchtdioden, so zu reflektieren, daß die Strahlen in einem begrenzten Bereich des Kanals oder Leitung verbleiben. Die doppelte Reflexion in den Spiegelpaaren, die in 90° Winkel zueinander stehen, schickt den axial gerichteten Anteil der Strahlung immer zurück, eben dorthin wo die Strahlung herkommt; der seitliche Anteil der Strahlung führt nur zu einem seitlichem Versatz im Kanal. Auf dieser Weise ist es sichergestellt, daß die Strahlung nur in einem eng begrenztem Bereich der Leitung oder Kanals bleibt, wo sie um so größere Intensität annimmt, je größer der Reflexionsgrad der Spiegel ist. Wenn man diese Kanäle mit einem für die Strahlung transparentem Fluid füllt, wie z.B. Luft für die UV-Strahlung, kann eine relativ schwache Lichtquelle die Strahlungsdichte im Kanal so hohe Werte annehmen lassen, daß es für die gewünschten Zweck - meistens Desinfektion - vollkommen ausreicht. In den meisten Fällen wird ein rechteckiger Querschnitt der Leitungen gewählt, aber auch andere, wie polygonale oder runde Profile sind anwendbar. Um eine ungehinderte Reflexion der Strahlung zu ermöglichen, sind metallische Spiegel ohne Abdeckung oder Schutzschicht erforderlich. Da der Effekt der doppelten Reflexion unabhängig von der Größe der Spiegel ist, können sehr kleine und längliche Spiegel-Segmente verwendet werden, was zu einer leichten Herstellung von derartigen Verspiegelungen von Kanälen / Leitungen führt. Der meistens störende Effekt der Divergenz beim Aussenden der Strahlung ist bei dieser Art der multiplen Reflektion unerheblich. Da die Strahlungsdichte bei Anwendung dieser Vorrichtung, abhängig vom Reflexionsgrad der Verspiegelung, bedeutend hohe Werte erreicht, die ein vielfaches der Strahlungsstärke der bloßen Lichtquelle übertrifft, kann diese als Licht Amplifizierer mittels Reflexionen im lokalen Areal, oder abgekürzt LARLA bezeichnet werden.

Ein Ausführungsbeispiel der erfindungsgemäßen Vorrichtung ist in den Zeichnungen dargestellt und wird im Folgenden näher erläutert.

Es zeigt:

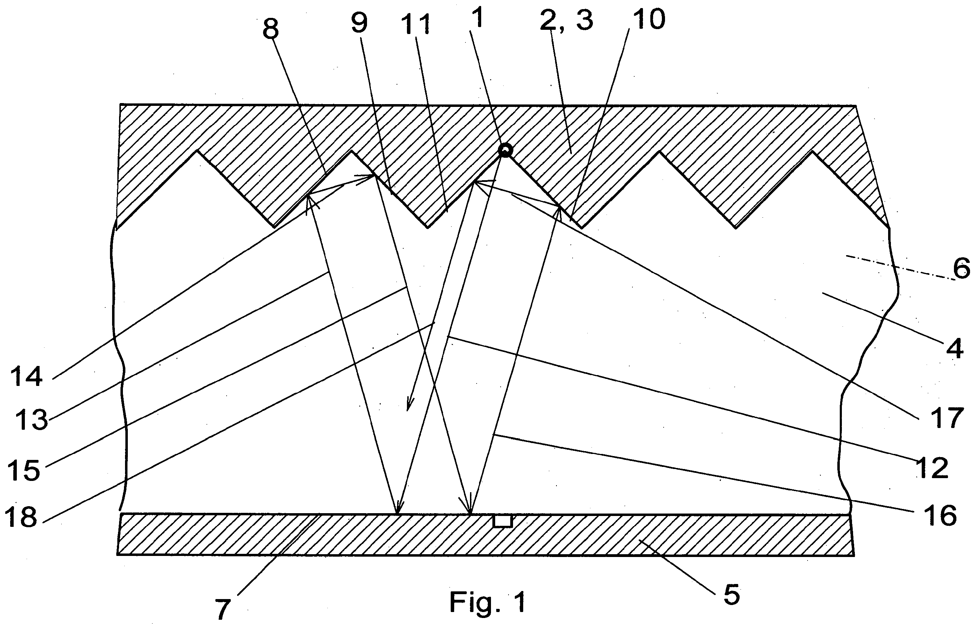

Fig. 1 Längsschnitt durch die Vorrichtung
Fig. 2 Querschnitt durch die Vorrichtung

Die vorliegende Erfindung bezieht sich auf eine Vorrichtung zur Lichtverstärkung mittels Reflexion in Leitungen mit etwa rechteckigen Querschnitt, wobei die Wände verspiegelt sind und versehen mit regelmäßig angeordneten Lichtquellen 1, dergestalt, daß die Lichtstrahlen mehrfach von den Wänden reflektiert werden.

Die Verstärkung des Lichts von einer quasi punktuellen Lichtquelle 1 erfolgt durch mehrfache spezielle Reflektionen in einem begrenzten Bereich einer Leitung 3 für den Transport von Fluiden, die für das verwendete Licht transparent sind, indem eine spezielle Gestaltung oder Geometrie der verspiegelten Leitungswänden 2, 4, 5, 6 verwendet wird, welche die Lichtstrahlen zwingen mehrheitlich immer denselben Weg zu nehmen und eine wiederholte Beleuchtung desselben Raumes oder Flächen bewirken, so daß eine Verflüchtigung der Strahlung entlang der Leitung 3 verhindert wird. Die Reflexionseigenschaften der Wände sind unterschiedlich gewählt und zwar dergestalt, daß nur eine Wand die Strahlen tendenziell in die Richtung woher sie kommen schickt, während die anderen Wände die Strahlen quasi normal reflektieren und zwar mit dem Effekt, daß keine Strahlen einen abgegrenzten Bereich der Leitung 3 verlassen können.

Die Strahlungsdichte abhängig vom Reflexionsgrad der Verspiegelung erreicht bedeutend hohe Werte, die ein vielfaches der Strahlungsstärke der bloßen Lichtquelle übertrifft und somit die Vorrichtung als **Licht Amplifizierer mittels Reflexionen im lokalen Areal,** oder abgekürzt LARLA bezeichnet werden kann. Ein beliebiger Strahl 12, befindlich in einer axialen Ebene die zugleich rechtwinklig zu den Wänden 2, 5 ist, einer Lichtquelle 1 vorhanden in der Wand 2 der Leitung 3 fällt auf eine Spiegelfläche 7 der Wand 5 und wird als Strahl 13 reflektiert und fällt auf eine Spiegelfläche 8, die Teil eines Doppel-Spiegelpaares 8, 9 ist, von wo er als Strahl 14 auf die andere Hälfte des Spiegelpaares 9 fällt, um von dort als Strahl 15 parallel zum Strahl 13 zurück zur Wand 5 zu gehen.

Der Strahl 15 von der Spiegelfläche 7 der Wand 5 geht als Strahl 16 parallel zum ersten Strahl 12 zurück zur Wand 2, um von der dortigen Doppel-Spiegelfläche 10,. 11 als Strahl 17 und danach als Strahl 18 zur Wand 5 geschickt zu werden, wobei der Strahl 18 nicht nur parallel zum ersten Strahl 12 ist, sondern auch sehr nahe an ihm gelegen ist, so daß ab diesem Zeitpunkt das Spiel der Reflektionen wie bereits beschrieben sich wiederholt. Dabei ist ersichtlich, daß die Strahlen den abgegrenzten Bereich nicht verlassen können.

Auch andere Strahlen der Lichtquelle 1 die nicht in normaler Ebene zu den Wänden 2, 5 sind, mit zusätzlichen seitlichen Reflektionen an den Wänden 4, 6 erfahren letztendlich eine ähnlich begrenzte Ausbreitung im Raum der Leitung 3, wie die vorhin analysierten Strahlen.

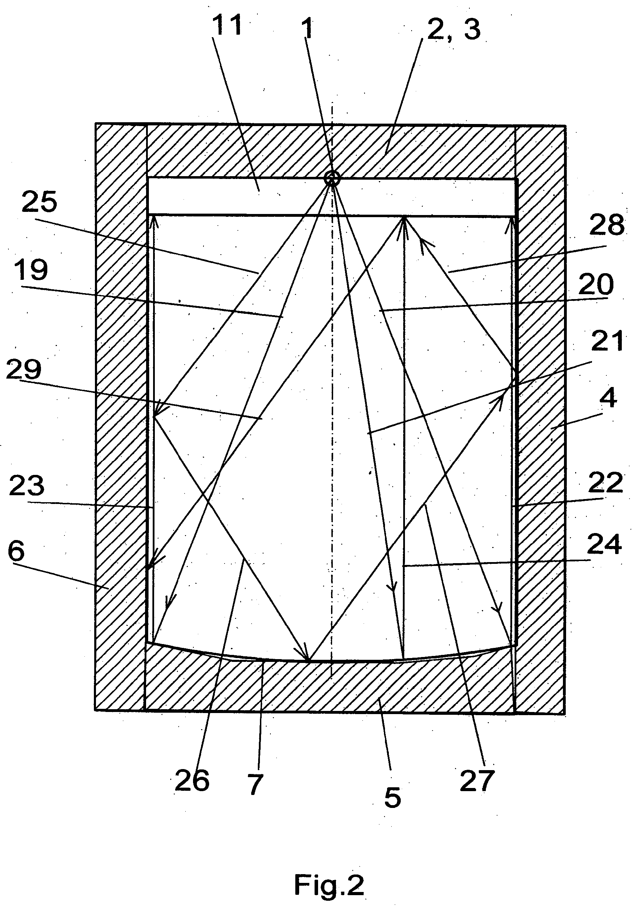

In einer Ebene quer auf die Leitung 3, wie ersichtlich in Fig. 2, die Verspiegelung 7 der Wand 5 bewirkt eine Konzentration der divergierenden Strahlen19, 20, 21, so daß diese quasi parallel zu den Wänden 4, 6 zu der Verspiegelung der Wand 2 gehen, um von dort wiederum zu der Wand 5 zu gehen, von wo sie etwas gebündelt in Richtung der Lichtquelle 1 zu gehen, von wo der Weg der Strahlen, wie bereits beschrieben erneut stattfindet.

Die Strahlen außerhalb des Kegels markiert von den Strahlen 19, 20 erfahren zusätzliche Reflexionen an den Wänden 4, 6, aber verbleiben im abgegrenzten Bereich.

Eine Innenwand einer quasi rechteckigen Leitung 3 ist mit jeweils einer Reihe von parallel angeordneten Spiegelpaaren 8, 9, ... belegt, wobei diese Spiegelpaare aus einzelnen länglichen Spiegeln im 90° Winkel zueinander bestehen, die zudem in 45° Winkel zur Leitungsachse stehen.

Eine kleinflächige Lichtquelle 1 ist im Tal zwischen den Spiegel-Elementen eines geeigneten Spiegelpaares plaziert, oder wenn sie außerhalb der Spiegelebene plaziert ist, wird durch einen Durchbruch von geringer räumlicher Ausdehnung ihre Strahlung in die Leitung hineingeschickt.

Die Seitenflächen 4, 6 der Leitung 3 sind meistens glatt und verspiegelt, während die Wand 5, die gegenüber der Wand 2 mit den Doppel-Spiegelpaaren ist, nach der Längsachse der Leitung 2 eine Zylindrizität aufweist, mit dem Radius etwa doppelt so groß wie der Abstand zwischen der Wand 2 und 5.

In einem anderen Ausführungsbeispiel der Erfindung wird statt einem rechteckigen, ein polygonaler oder gar runder Querschnitt der Leitung 3 eingesetzt, wobei die Eigenschaft der begrenzten axialen Ausbreitung der Strahlen in etwa erhalten bleibt. Alle verwendeten Spiegelpaare sind in der Regel metallische Spiegel ohne Abdeckung oder Schutzschicht.

Vorrangig werden relativ sehr kleine und längliche Spiegel-Segmente verwendet, was zu einer leichten Herstellung von derartigen Verspiegelungen der Leitung 3 führt.

## Patentansprüche

1. Leitung mit Lichtverstärkung mittels Reflexion mit etwa rechteckigem Querschnitt der Leitung, wobei die Wände der Leitung verspiegelt sind und versehen mit regelmäßig angeordneten Lichtquellen (1), dergestalt, daß die Lichtstrahlen mehrfach von den Wänden reflektiert werden,
wobei
a. eine Verstärkung des Lichts von einer quasi punktuellen Lichtquelle (1) durch mehrfache spezielle Reflektionen in einem begrenzten Bereich der Leitung (3) für den Transport von Fluiden, die für das verwendete Licht transparent sind, stattfindet, indem eine spezielle Gestaltung oder Geometrie der verspiegelten Leitungswänden (2, 4, 5, 6) verwendet wird, welche die Lichtstrahlen zwingen mehrheitlich immer denselben Weg zu nehmen und eine wiederholte Beleuchtung desselben Raumes oder Flächen bewirken, so daß eine Verflüchtigung der Strahlung entlang der Leitung (3) verhindert wird,
b. wobei die Reflexionseigenschaften der Wände unterschiedlich gewählt sind und zwar dergestalt, daß nur eine Wand die Strahlen tendenziell in die Richtung woher sie kommen schickt, während die anderen Wände die Strahlen quasi normal reflektieren und zwar mit dem Effekt, daß keine Strahlen einen abgegrenzten Bereich der Leitung (3) verlassen können,
c. wobei die Strahlungsdichte abhängig vom Reflexionsgrad der Verspiegelung bedeutend hohe Werte erreicht, die ein vielfaches der Strahlungsstärke der bloßen Lichtquelle übertrifft.

2. Leitung nach Anspruch 1,
**dadurch gekennzeichnet, daß**
a. ein beliebiger Strahl (12), befindlich in einer axialen Ebene die zugleich rechtwinklig zu den Wänden (2, 5) ist, einer Lichtquelle (1) vorhanden in der Wand (2) der Leitung (3) auf eine Spiegelfläche (7) der Wand (5) fällt und als Strahl (13) reflektiert wird und auf eine Spiegelfläche (8) fällt, die Teil eines Doppel-Spiegelpaares (8, 9) ist, von wo er als Strahl (14) auf die andere Hälfte des Spiegelpaares (9) fällt, um von dort als Strahl (15) parallel zum Strahl (13) zurück zur Wand (5) zu gehen,
b. wobei der Strahl (15) von der Spiegelfläche (7) der Wand (5) als Strahl (16) parallel zum ersten Strahl (12) zurück zur Wand (2) geht, um von der dortigen Doppel-Spiegelfläche (10, 11) als Strahl (17) und danach als Strahl (18) zur Wand (5) geschickt zu werden, wobei der Strahl (18) nicht nur parallel zum ersten Strahl (12) ist, sondern auch sehr nahe an ihm gelegen ist, so daß ab diesem Zeitpunkt das Spiel der Reflektionen wie bereits beschrieben sich wiederholt; wobei die Strahlen den abgegrenzten Bereich nicht verlassen können;
c. wobei auch andere Strahlen der Lichtquelle (1) die nicht in normaler Ebene zu den Wänden (2, 5) sind, mit zusätzlichen seitlichen Reflektionen an den Wänden (4, 6) letztendlich eine ähnlich begrenzte Ausbreitung im Raum der Leitung (3) erfahren, wie die Strahlen (15) bis (18);
d. wobei in einer Ebene quer zur Leitung (3) die Verspiegelung (7) der Wand (5) eine Konzentration von divergierenden Strahlen (19, 20, 21) bewirkt, so daß diese quasi parallel zu den Wänden (4, 6) zu der Verspiegelung der Wand (2) gehen, um von dort wiederum zu der Wand (5) zu gehen, von wo sie etwas gebündelt in Richtung der Lichtquelle (1) zu gehen, von wo der Weg der Strahlen erneut stattfindet,
e. wobei die Strahlen außerhalb des Kegels markiert von den Strahlen (19, 20) zusätzliche Reflexionen an den Wänden (4, 6) erfahren, aber im abgegrenzten Bereich verbleiben.

3. Leitung nach Anspruch 1 und 2,
**dadurch gekennzeichnet, daß**
a. eine Innenwand einer quasi rechteckigen Leitung (3) mit jeweils einer Reihe von parallel angeordneten Spiegelpaaren (8, 9) belegt ist, wobei diese Spiegelpaare aus einzelnen länglichen Spiegeln in 90° Winkel zueinander bestehen, die zudem im 45° Winkel zur Leitungsachse stehen,
b. wobei eine kleinflächige Lichtquelle (1), welche auch Teil einer Reihe von solchen regelmäßig angebrachten Lichtquellen sein kann, im Tal zwischen den Spiegel-Elementen eines geeigneten Spiegelpaares plaziert ist, oder wenn sie außerhalb der Spiegelebene plaziert ist, durch einen Durchbruch von geringer räumlicher Ausdehnung ihre Strahlung in die Leitung (3) hinein schickt,
c. wobei die Seitenflächen (4, 6) der Leitung (3) meistens glatt und verspiegelt sind, während die Wand (5), die gegenüber der Wand (2) mit den Doppel-Spiegelpaaren ist, zu der Längsachse der Leitung (3) eine Zylindrizität aufweist, mit einem Radius etwa doppelt so groß wie der Abstand zwischen der Wand (2) und (5).

4. Leitung nach Anspruch 3
**dadurch gekennzeichnet, daß**
ein näherungsweise rechteckiger und polygonaler oder gar teilgerundeter Querschnitt der Leitung (3) eingesetzt wird, wobei die Eigenschaft der begrenzten axialen Ausbreitung der Strahlen in etwa erhalten bleibt.

5. Leitung nach einem der Ansprüche 2 bis 4,
**dadurch gekennzeichnet, daß**
a. alle verwendeten Spiegelpaare in der Regel metallische Spiegel ohne Abdeckung oder Schutzschicht sind;
b. wobei vorrangig relativ sehr kleine und längliche Spiegel-Segmente verwendet werden, was zu einer leichten Herstellung von derartigen Verspiegelungen der Leitung (3) führt.

6. Leitung nach einem der Ansprüche 1 bis 5,
**dadurch gekennzeichnet, daß**
für die Anwendung eine Leitung mit Luft gefüllt wird, um eine wirksame Desinfektion mit Hilfe einer oder einiger wenigen schwachen UV-Lichtquellen zu erreichen.

## Claims

1. Conduit for amplifying light by means of reflection in conduits with an approximately rectangular cross-section, the walls being mirrored and provided with regularly arranged light sources (1), such that the light rays are reflected several times by the walls, **characterized in that**
a. an amplification of the light from a quasi-point light source (1) by multiple special reflections in a limited area of a conduit (3) for the transport of fluids, that are transparent to the light used, takes place by a special design or geometry of the mirrored conduit walls (2, 4, 5, 6) is used, which force the light rays to mostly always take the same path and cause repeated illumination of the same room or area, so that the radiation is prevented from disappeare along the conduit (3),
b. The reflection properties of the walls are chosen differently in such a way, that only one wall tends to send the rays in the direction from which they come, while the other walls reflect the rays more or less normally, with the effect that no rays of a defined area of the conduit (3) can leave it,
c. whereby the radiation density, depending on the degree of reflection of the mirror coating, reaches significantly high values, which exceeds the radiation intensity of the mere light source many times over.

2. Conduit according to claim 1, **characterized in that**
a. any beam (12), located in an axial plane, which is also perpendicular to the walls (2, 5), of a light source (1) present in the wall (2) of the conduit (3) on a mirror surface (7) of the wall (5) falls and is reflected as a beam (13) and falls on a mirror surface (8), which is part of a double mirror pair (8, 9), from where it falls as a beam (14) onto the other half of the mirror pair (9 ) falls, in order to go from there as a beam (15) parallel to the beam (13) back to the wall (5), 6
b. wherein the beam (15) goes from the mirror surface (7) of the wall (5) as a beam (16) parallel to the first beam (12) back to the wall (2) in order to be reflected from the double mirror surface (10, 11) as beam (17) and then as a beam (18) to be sent to the wall (5), wereby the beam (18) not only being parallel to the first beam (12), but also being very close to it, so that from this moment the game of reflections repeats itself, as already described; whereby the rays cannot leave the demarcated area;
c. whereby other rays from the light source (1), which are not in a normal plane to the walls (2, 5,) ultimately experience a similarly limited propagation in the space of the conduit (3) with additional lateral reflections on the walls (4, 6), like rays (15) to (18);
d. wherein in a plane transverse to the conduit (3), the mirroring (7) of the wall (5) causes a concentration of diverging rays (19, 20, 21), so that they go quasi-parallel to the walls (4, 6) to the mirroring the wall (2), in order to go from there again to the wall (5), from where they go somewhat bundled towards the light source (1), from where the path of the rays takes place again,
e. wherein the rays outside the cone, marked by the rays (19, 20), experience additional reflections on the walls (4, 6), but remain in the delimited area.

3. Conduit according to claims 1 and 2, **characterized in that**
a. an inner wall side of a quasi-rectangular conduit (3) is covered with a row of mirror pairs (8, 9) arranged in parallel, these mirror pairs consisting of individual elongated mirrors at 90° angles to one another, which are also at a 45° angle to the conduit axis,
b. wherein a small-area light source (1), which can also be part of a series of such regularly installed light sources, in the valley which is placed between the mirror elements of a suitable pair of mirrors, or if it is placed outside the mirror plane, sends its radiation into the conduit (3) through a breakthrough of small spatial extent,
c. wherein the side surfaces (4, 6) of the conduit (3) are mostly smooth and mirrored, while the wall (5), which is opposite the wall (2) with the double mirror pairs, has a cylindricity to the longitudinal axis of the conduit (3), which cylindricity has a radius approximately twice as large as the distance between the wall (2) and (5).

4. Conduit according to claim 3, **characterized in that** an approximately rectangular and polygonal or even partially rounded cross section of the conduit (3) is used, the property of the limited axial spread of the beams being approximately retained.

5. Conduit according to one of claims 2 to 4, **characterized in that**
a. all pairs of mirrors used are usually metallic mirrors without a cover or protective layer;
b. wherein relatively very small and elongated mirror segments are primarily used, which leads to easy production of such mirror coatings on the conduit (3).

6. Conduit according to one of claims 1 to 5, **characterized in that** for use a conduit which is filled with air in order to achieve effective disinfection with the aid of one or a few weak UV light sources.

## Revendications

1. Conduit avec amplification de la lumière par réflexion, de section transversale approximativement rectangulaire, les parois du conduit étant réfléchissantes et pourvues de sources lumineuses (1) disposées régulièrement, de telle sorte que les rayons lumineux sont réfléchis plusieurs fois par les parois,
où
a. une amplification de la lumière provenant d'une source de lumière quasi ponctuelle (1) est réalisée par des réflexions spéciales multiples dans une zone limitée du conduit (3) pour le transport de fluides qui sont transparents à la lumière utilisée, en utilisant une conception ou une géométrie spéciale des parois réfléchissantes du conduit (2, 4, 5, 6) qui obligent les rayons lumineux à suivre majoritairement toujours le même chemin et provoquent un éclairage répété du même espace ou des mêmes surfaces, de sorte qu'une volatilisation du rayonnement le long du conduit (3) est empêchée,
b. les propriétés de réflexion des parois sont choisies différemment, de telle sorte qu'une seule paroi tend à envoyer les rayons dans la direction d'où ils viennent, tandis que les autres parois réfléchissent les rayons de manière quasi normale, avec pour effet qu'aucun rayon ne peut quitter une zone délimitée du conduit (3),
c. la densité du rayonnement atteint, en fonction du degré de réflexion de la couche réfléchissante, des valeurs considérablement élevées qui dépassent plusieurs fois l'intensité du rayonnement de la source lumineuse nue.

2. Conduit selon la revendication 1,
**caractérisé en ce que**
a. un rayon quelconque (12), situé dans un plan axial qui est en même temps perpendiculaire aux parois (2, 5), d'une source lumineuse (1) présente dans la paroi (2) du conduit (3) tombe sur une surface réfléchissante (7) de la paroi (5) et est réfléchi comme rayon (13) et tombe sur une surface réfléchissante (8), qui fait partie d'une double paire de miroirs (8, 9), d'où il tombe en tant que rayon (14) sur l'autre moitié de la paire de miroirs (9), d'où il revient en tant que rayon (15) parallèle au rayon (13) vers la paroi (5),
b. le rayon (15) partant de la surface réfléchissante (7) de la paroi (5) et revenant vers la paroi (2) en tant que rayon (16) parallèle au premier rayon (12), pour être envoyé par la double surface réfléchissante (10, 11) qui s'y trouve en tant que rayon (17), puis en tant que rayon (18) vers la paroi (5), le rayon (18) étant non seulement parallèle au premier rayon (12), mais aussi très proche de celui-ci, de sorte qu'à partir de ce moment, le jeu des réflexions se répète comme décrit précédemment; les rayons ne pouvant pas quitter la zone délimitée ;
c. les autres rayons de la source lumineuse (1) qui ne sont pas dans le plan normal des parois (2, 5), avec des réflexions latérales supplémentaires sur les parois (4, 6), finissent par se propager dans l'espace du conduit (3) de manière aussi limitée que les rayons (15) à (18) ;
d. dans un plan transversal au conduit (3), le miroitement (7) de la paroi (5) provoque une concentration de rayons divergents (19, 20, 21), de sorte que ceux-ci se dirigent quasiment parallèlement aux parois (4, 6) vers le miroitement de la paroi (2), pour de là se diriger à nouveau vers la paroi (5), d'où ils se dirigent, quelque peu concentrés, vers la source de lumière (1), d'où le trajet des rayons a de nouveau lieu,
e. les rayons à l'extérieur du cône, marqués par les rayons (19, 20), subissent des réflexions supplémentaires sur les parois (4, 6), mais restent dans la zone délimitée.

3. Conduit selon les revendications 1 et 2,
**caractérisé en ce que**
a. une paroi intérieure d'un conduit quasi rectangulaire (3) est recouverte à chaque fois d'une série de paires de miroirs (8, 9) disposés parallèlement, ces paires de miroirs étant constituées de miroirs allongés individuels formant un angle de 90° les uns par rapport aux autres, qui forment en outre un angle de 45° par rapport à l'axe du conduit,
b. une source lumineuse (1) de petite surface, qui peut également faire partie d'une série de telles sources lumineuses disposées régulièrement, est placée dans la vallée entre les éléments de miroir d'une paire de miroirs appropriée ou, si elle est placée en dehors du plan des miroirs, envoie son rayonnement dans le conduit (3) par une ouverture de faible extension spatiale,
c. les faces latérales (4, 6) du conduit (3) étant le plus souvent lisses et réfléchissantes, tandis que la paroi (5) opposée à la paroi (2) avec les paires de miroirs doubles présente une cylindricité par rapport à l'axe longitudinal du conduit (3), avec un rayon environ double de la distance entre la paroi (2) et (5).

4. Conduit selon la revendication 3
**caractérisé en ce que**
on utilise une section approximativement rectangulaire et polygonale, voire partiellement arrondie, de la conduite (3), tout en conservant approximativement la propriété de propagation axiale limitée des rayons.

5. Conduit selon l'une quelconque des revendications 2 à 4,
**caractérisé en ce que**
a. toutes les paires de miroirs utilisées sont généralement des miroirs métalliques sans revêtement ou couche de protection ;
b. des segments de miroirs relativement très petits et allongés étant utilisés en priorité, ce qui conduit à une fabrication aisée de tels miroirs de la ligne (3).

6. Conduit selon l'une quelconque des revendications 1 à 5,
**caractérisé en ce que**
pour l'application, un conduit est rempli d'air afin d'obtenir une désinfection efficace à l'aide d'une ou de quelques sources de lumière UV de faible intensité.
